**Europäisches Patentamt**

**European Patent Office**

**Office européen des brevets**

(19)

(11) Veröffentlichungsnummer: **0 410 272 A1**

# (12) EUROPÄISCHE PATENTANMELDUNG

(21) Anmeldenummer: **90113651.5**

(22) Anmeldetag: **17.07.90**

(51) Int. Cl.5: **C07K 3/02**, //A61K39/395

(30) Priorität: **24.07.89 DE 3924420**

(43) Veröffentlichungstag der Anmeldung:
**30.01.91 Patentblatt 91/05**

(84) Benannte Vertragsstaaten:
**AT BE CH DE DK ES FR GB GR IT LI LU NL SE**

(71) Anmelder: **BIOTEST PHARMA GMBH**
**Landsteiner Strasse 5**
**D-6072 Dreieich(DE)**

(72) Erfinder: **Stephan, Wolfgang, Dr., Dipl.-Chem.**
**Philipp-Holzmann Strasse 84**
**D-6072 Dreieich(DE)**
Erfinder: **Dichtelmüller, Herbert, Dr.**
**Rossertstr. 14**
**D-6231 Sulzbach(DE)**

(74) Vertreter: **Beil, Hans Chr., Dr. et al**
**Beil, Wolff und Beil, Rechtsanwälte**
**Adelonstrasse 58 Postfach 80 01 40**
**D-6230 Frankfurt am Main 80(DE)**

(54) **Verfahren zur Herstellung eines Immunglobulin-Präparates aus Kolostrum.**

(57) Verfahren zur Herstellung eines Immunglobulin-Präparates aus Kolostrum

Verfahren zur Herstellung eines Immunglobulin-Präparates aus Kolostralmilch von nicht immunisierten Säugern der ersten 30 Stunden post partum mit den Verfahrensschritten: Verdünnen mit destilliertem Wasser, Abtrennen des Fettes, Pasteurisieren, Sterilisieren, z.B. durch Bestrahlen mit UV-Licht oder Behandeln mit TNBP,und Lyophilisieren oder Sprühtrocknen. Vor oder nach der Sterilisation ist ein Ankonzentrieren durch Ultrafiltration möglich.

EP 0 410 272 A1

## VERFAHREN ZUR HERSTELLUNG EINES IMMUNGLOBULIN-PRÄPARATES AUS KOLOSTRUM

Die Erfindung betrifft ein Verfahren zur Isolierung, Sterilisierung und Stabilisierung von Antikörpern aus Kolostrum nicht immunisierter Säuger in hohen Ausbeuten. Die Ausbeuten betragen rund das Vierfache im Vergleich zu herkömmlichen Methoden.

Zahlreiche Verfahren zur Gewinnung von Immunglobulinen aus Milch oder Kolostralmilch sind bisher bekannt.

Beispielsweise wird in der US-PS 4 526 715 ein Verfahren zur Bereitstellung von Immunglobulinen aus Milch über verschiedene chromatographische Trennungsschritte beschrieben. Die US-PS 3 128 230 betrifft ein Verfahren zur Herstellung eines aus Milch erhältlichen Präparates zur Behandlung verschiedener bakterieller Infektionen. In der US-PS 4 265 925 und der US-PS 1 573 995 werden Verfahren zur Herstellung von Proteinkonzentraten aus Lactoserum beschrieben, wobei diese Proteine aufgrund einer Hitzebehandlung grösstenteils denaturiert werden. Schliesslich werden in der DE-OS 28 13 284 sowie in der EP-A 0 046 909 und der EP-A 0 064 103 Kolostralmilchaufbereitungsverfahren beschrieben, wobei Immunglobulin enthaltende Produkte als entzündungshemmende Mittel erhalten werden.

Bei der Herstellung pharmakologisch wirksamer immunglobulinhaltiger Präparate nach den obengenannten Verfahren wird von Milch bzw. Kolostralmilch hyperimmunisierter Säuger, insbesondere Rinder, ausgegangen. Hierbei müssen also, um eine antibakterielle oder antivirale Effizienz gegen bestimmte Krankheitserreger zu erzielen, den Tieren zunächst ein oder mehrere einen Antikörper bzw. eine Gruppe spezifischer Antikörper induzierende Bakterienstämme bzw. deren Antigene injiziert werden. Die Wirksamkeit der so gewonnenen Präparate entspricht der Art und Anzahl der verabreichten Bakterienstämme.

Auch zur Bereitstellung eines aus Milch erhältlichen Präparates mit einer spezifischen Antikörperaktivität (z.B. gegen bakterielle Antigene) wird zunächst der Säuger mit speziellen Antigenen immunisiert. Die dann gewonnene Milch weist einen hohen Antikörpertiter gegen das spezielle Antigen auf, wobei die Milch als Trägermedium dient.

Darüberhinaus kann ein Immunglobulin-enthaltendes Präparat aus dem Kolostrum nicht immunisierter Säuger durch Tangentialfiltration gemäss DE-PS 34 32 718 hergestellt werden.

Ferner wird in der EP-A 89 104 016.4 ein Immunglobulin-Präparat aus dem Kolostrum nicht immunisierter Säuger unter anderem zur Prophylaxe und Therapie von Durchfallerkrankungen beschrieben.Bei diesem Verfahren wird nach dem Entfernen der Fettphase das Casein im sauren pH-Bereich entfernt. Dieser Schritt ist stark ausbeutereduzierend. Überraschenderweise wurde gefunden, dass man ein Immunglobulin-Präparat aus Kolostrum erhalten kann, das nach Entfernung der Fettphase ohne jede weitere Fraktionierung praktisch die gleiche Antikörperkonzentration enthält, wie die Produkte, die nach dem beschriebenen Verfahren unter Casein-Entfernung hergestellt werden.

Da der Säureschritt, der bei der Casein-Entfernung stark Virus- und Bakterien-eliminierend wirkt, im erfindungsgemäßen Verfahren wegfällt, muss die Magermilch aus Kolostrum zur Virus- und Bakterieninaktivierung nach an sich bekannten Verfahren, wie beispielsweise durch Bestrahlen mit UV-Licht oder Tri-n-Butylphosphat-Behandlung sterilisiert werden.

Der virusinaktivierende Effekt von UV-Licht an dieser Stelle war ebenfalls überraschend, da infolge der starken Trübung der Kolostralmagermilch eine schlechte Absorption der wirksamen UV-Bestrahlung angenommen werden musste.

Das nach dem neuen Verfahren hergestellte Immunglobulin-Präparat kann durch Ultrafiltration ankonzentriert und durch Lyophilisation bzw. Sprühtrocknung stabilisiert werden. Das Ankonzentrieren durch Ultrafiltration kann dabei vor oder nach der Sterilisation erfolgen.

Beispiel 1

100 kg Kolostralmilch von Rind wurden mit $H_2O$ 1:3 verdünnt, entfettet und pasteurisiert (70 °C/20 Sek.). 30 kg der so gewonnenen Kolostralmagermilch wurden ultrafiltriert, mit 4 x 20 Watt UV-bestrahlt und sprühgetrocknet (Präparat I).

Die restliche Menge (270 kg) wurde gemäss dem Verfahren der EP-A 89 104 016.4 durch Säuerung (Caseinabtrennung), Ultrafiltration und Sprühtrocknung aufgearbeitet (Präparat II). Die Analyse der beiden Präparate zeigt die nachfolgende Tabelle 1.

Tabelle 1

|  | Präparat I (erfindungsgemäss) | Präparat II (Vergleich) |
|---|---|---|
| Protein (g/100 ml) | 7,9 | 7,7 |
| pH | 6,1 | 4,7 |
| IgG (mg/100 ml) | 4900 | 5800 |
| IgM (mg/100 ml) | 1800 | 1900 |
| Lipide (mg/100 ml) | 180 | <100 |
| Lactose (mg/100 ml) | 416 | 650 |
| E.coli (rezipr.Titer) | 640 | 1280 |
| Staph.aur.(rezipr.Titer) | 320 | 320 |
| Ausbeute (kg/100 kg) | 18,5 | 5,5 |

Beispiel 2

100 kg Kolostralmilch wurden mit $H_2O$ 1:1 verdünnt, entfettet und pasteurisiert. Danach wurde mit 2 x 20 Watt UV-bestrahlt, ultrafiltriert und das Präparat lyophilisiert. Die Eigenschaften dieses Präparates entsprechen denjenigen des Beispiels 1.

Der Umfang der Bakterieninaktivierung durch die erfindungsgemässe Verfahrensweise ist in der nachfolgenden Tabelle 2 dargestellt.

Tabelle 2

| Fraktion | Keime/ml | Inaktivierung ($\log_{10}\downarrow$) |
|---|---|---|
| Kolostrum | $>2 \times 10^7$ | $>3,0$ |
| Magermilch (M) | $1,4 \times 10^4$ | 1,9 |
| M + UV | $1,6 \times 10^2$ | |
| | | $\overline{4,9}$ |

Das Ausmass der Virusinaktivierung durch die UV-Bestrahlung ist in der nachfolgenden Tabelle 3 dargestellt.

Tabelle 3

| | Virustyp | | | |
|---|---|---|---|---|
| Fraktion | øx 171 | | Kappa | |
| | Phagen/ml | Inakt. ($\log_{10}\downarrow$) | Phagen/ml | Inakt. ($\log_{10}\downarrow$) |
| Vor UV | $6,2 \times 10^7$ | | $3,7 \times 10^8$ | |
| Nach UV | $3,9 \times 10^2$ | 5,2 | $8,6 \times 10^2$ | 5,6 |

Beispiel 3

100 l Kolostralmilch wurden mit Wasser 1:1 verdünnt, entfettet und pasteurisiert. Danach wurde mit 2 l Tri-n-butylphosphat (TNBP) behandelt, ultrafiltriert und lyophilisiert. Die Eigenschaften dieses Präparats entsprechen denjenigen des Beispiels 1.

**Ansprüche**

1. Verfahren zur Herstellung eines Immunglobulin-Präparates aus Kolostralmilch von nicht immunisierten Säugern der ersten 30 Stunden post partum, dadurch gekennzeichnet, dass man
   a) die Kolostralmilch mit destilliertem Wasser verdünnt,
   b) das Fett abtrennt,
   c) die entfettete Kolostralmilch pasteurisiert,
   d) sterilisiert und
   e) lyophilisiert oder sprühtrocknet.
2. Verfahren nach Anspruch 1, dadurch gekennzeichnet, dass die Sterilisation durch Bestrahlen mit UV-Licht erfolgt.
3. Verfahren nach Anspruch 1, dadurch gekennzeichnet, dass die Sterilisation durch Behandeln mit Tri-n-butylphosphat erfolgt
4. Verfahren nach einem der Ansprüche 1 bis 3, dadurch gekennzeichnet, dass man Kolostralmilch der ersten 10 Stunden post partum verwendet.
5. Verfahren nach einem der Ansprüche 1 bis 4, dadurch gekennzeichnet, dass man das Präparat vor der UV-Bestrahlung durch Ultrafiltration ankonzentriert.
6. Verfahren nach einem der Ansprüche 1 bis 4, dadurch gekennzeichnet, dass man das Präparat nach der UV-Bestrahlung durch Ultrafiltration ankonzentriert.

4

**Europäisches**
**Patentamt**

**EUROPÄISCHER**
**RECHERCHENBERICHT**

Nummer der Anmeldung

**EP 90 11 3651**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-4 051 235  (R.R. PLYMATE)<br>* Ansprüche; Spalte 1, Zeilen 49-52; Spalte 2, Zeilen 22-24 *<br>- - - | 1-6 | C 07 K 3/02 //<br>A 61 K 39/395 |
| A | PATENT ABSTRACTS OF JAPAN, Band 9, Nr. 211 (C-300)[1934], 29. August 1985;<br>& JP-A-60 75 433 (ZENKOKU NOUGIYOU KIYOUDOUKU-MIAI RENGOUKAI) 27-04-1985<br>* The entire abstract *<br>- - - - - | 1-6 | |

| | RECHERCHIERTE SACHGEBIETE (Int. Cl.5) |
|---|---|
| | C 07 K<br>A 61 K |

Der vorliegende Recherchenbericht wurde für alle Patentansprüche erstellt

| Recherchenort | Abschlußdatum der Recherche | Prüfer |
|---|---|---|
| Den Haag | 06 November 90 | RYCKEBOSCH A.O.A. |